(19) Europäisches Patentamt
European Patent Office
Office européen des brevets

(11) Numéro de publication: **0 214 053**
**A1**

(12) **DEMANDE DE BREVET EUROPEEN**

(21) Numéro de dépôt: **86401887.4**

(22) Date de dépôt: **27.08.86**

(51) Int. Cl.[4]: **G 01 N 33/545**
G 01 N 33/53, C 12 M 1/40

(30) Priorité: **30.08.85 FR 8512965**

(43) Date de publication de la demande:
**11.03.87 Bulletin 87/11**

(84) Etats contractants désignés:
**AT BE CH DE GB IT LI LU NL SE**

(71) Demandeur: **INSTITUT NATIONAL DE LA SANTE ET DE LA RECHERCHE MEDICALE (INSERM)**
**101, rue de Tolbiac**
**F-75654 Paris Cedex 13 (FR)**

**CENTRE NATIONAL DE LA RECHERCHE SCIENTIFIQUE (CNRS)**
**15, Quai Anatole France**
**F-75007 Paris (FR)**

(72) Inventeur: **Boitieux, Jean-Louis**
**Rue Jules Verne Jumel**
**F-80250 Ailly S/Noye (FR)**

**Thomas, Daniel**
**Domaine de Rimberlieu 33 Allée de l'Etang**
**F-60450 Villiers Sur Coudun (FR)**

(74) Mandataire: **Jolly, Jean-Pierre et al**
**Cabinet BROT et JOLLY 83, rue d'Amsterdam**
**F-75008 Paris (FR)**

(54) **Supports protéiques modifiés aptes à immobiliser de façon covalente ou réversible des anticorps ou des substances immunologiquement actives, et leurs applications au dosage et à la purification d'antigènes ou similaires.**

(57) L'invention concerne un support protéique modifié en gélatine de peau de porc adhérent à un film de matière plastique, apte à immobiliser de façon réversible une substance biologiquement active.

Cette gélatine comprend, à sa surface des sites accessibles constitués par des ligands aptes à se combiner de façon réversible avec un anti-corps ou une substance biologiquement active.

Le support sur lequel sont fixés des anticorps peut former une membrane à activité enzymatique que l'on peut combiner avec un capteur électro-chimique.

EP 0 214 053 A1

## Description

Supports protéiques modifiés aptes à immobiliser de façon covalente ou réversible des anticorps ou des substances immunologiquement actives, et leurs applications au dosage et à la purification d'antigènes ou similaires.

La présente invention concerne des supports protéiques modifiés aptes à immobiliser de façon réversible des anticorps ou des substances immunologiquement actives, marqués par une enzyme. L'invention concerne également diverses utilisations de ces supports notamment leur application au dosage et à la purification d'antigènes, d'haptènes ou, plus généralement, de substances immunologiquement actives dans les liquides biologiques ou autres.

On sait que, depuis plusieurs années, de nombreux dosages de substances présentes à faible concentration dans les liquides biologiques sont réalisés par des méthodes radio-immunologiques. L'utilisation des radio-isotopes pour le dosage de substances immunologiquement actives, bien que considérée comme la méthode la plus fiable actuellement, présente toutefois divers inconvénients, dont, en particulier, une précision médiocre et un manque de reproductibilité des résultats obtenus. Les radio-isotopes étant en outre coûteux et leur emploi sujet à diverses contraintes administratives, les milieux scientifiques ont été amenés, plus récemment, à étudier d'autres méthodes de dosage, faisant appel à l'activité catalytique des enzymes.

L'activité catalytique de ces macromolécules biologiques est, en effet, bien connue, la spécificité de cette activité pouvant être large, mais étant le plus souvent étroite. Le dosage de certaines substances à l'aide d'enzymes libres est d'ailleurs en usage depuis longtemps. C'est ainsi, par exemple, que l'on dose le glucose au moyen de glucose oxydase, qui le transforme en acide gluconique et en eau oxygénée, cette dernière oxydant un colorant qui se prête à une détermination colorimétrique. De même, l'acide glutamique peut être dosé à l'aide de la glutamate décarboxylase, qui produit du gaz carbonique, que l'on peut mettre en évidence par des moyens connus.

Des techniques plus récentes utilisent cependant l'activité enzymatique comme moyen de marquage de molécules intervenant dans des procédés d'analyse immunologique et l'on a ainsi envisagé de marquer des antigènes ou des anticorps non plus avec des radio-isotopes, mais avec des enzymes telles que la glucose oxydase, dont il est possible de doser facilement l'activité par colorimétrie. Ces techniques, dites d'enzymo-immunologie, ont suscité un vif intérêt, mais la cinétique colorimétrique de l'enzyme utilisée est souvent contrariée par certains composés présents dans le milieu biologique traité, de sorte que l'on a été amené à envisager des techniques physico-chimiques n'impliquant pas de contact direct avec le milieu biologique.

C'est ainsi qu'il a été proposé de fixer sur une membrane protéique artificielle des anticorps spécifiques d'un antigène déterminé, marqués avec une enzyme, en vue de relier l'activité enzymatique de la membrane ainsi modifiée à la concentration en antigène du liquide biologique étudié (voir S.L. BOITIEUX, G. DESMETS et D. THOMAS, "Evolution des capteurs biologiques en analyse clinique", Ann. Biol. clin., 1985, 43, 183-191). En partant des électrodes à glucose, qui associent une électrode à $pO_2$ et un film polyprotéique auquel est incorporée une enzyme spécifique du β glucose, la glucose oxydase, on a ainsi réalisé des électrodes enzymo-immunologiques, comprenant une électrode à $pO_2$ ou électrode de Clark ou tout autre capteur électrochimique (électrode à $H_2O_2$ ou électrodes spécifiques ou certains ions), sur la tête de laquelle est fixée une membrane protéique artificielle sur laquelle sont immobilisés des anticorps spécifiques d'un antigène à doser ou d'anticorps, couplés avec une enzyme.

Bien entendu, l'activité des enzymes est liée au maintien de l'intégrité de leur conformation tertiaire, en particulier au niveau de leur site actif, et leur mode d'immobilisation doit donc faire appel à des procédés doux et bien contrôlés.

On a donc envisagé, dans ce but, des méthodes d'immobilisation par inclusion dans le support, par adsorption ou liaison ionique, par liaison covalente ou par réticulation. Ces procédés conduisent toutefois à des supports protéiques auxquels l'enzyme est fixée de façon irréversible et, dans le cas de dosages faisant appel à des électrodes enzymo-immunologiques, il est par conséquent nécessaire de changer à chaque nouveau dosage la membrane porteuse d'activité enzymatique couplée à l'électrode. Cette sujétion complique donc les processus de dosage et en ralentit la cadence en s'opposant à leur automatisation complète.

La présente invention vise à remédier à cet inconvénient en proposant de nouveaux supports protéiques sur lesquels des anticorps puissent être immobilisés de façon réversible, de manière à pouvoir en être séparés aisément à la fin d'un dosage ou d'une série de dosages effectués à l'aide d'une électrode couplée à une membrane constituée d'un tel support.

L'invention a également pour but de proposer un procédé de dosage d'antigènes ou d'haptènes, virus, anticorps ou toute substance immunologiquement active, à l'aide d'une telle électrode, qui puisse être entièrement automatisé.

L'invention vise enfin a proposer un support protéique qui puisse être utilisé pour la purification à l'aide d'un même support de diverses susbtances immunologiquement actives.

A cet effet, l'invention a pour objet un support protéique modifié apte à immobiliser de façon réversible une substance biologiquement active, caractérisé en ce que ledit support comprend de la gélatine de peau de porc adhérant à un film d'une matière plastique ou similaire, apte à lui conférer une résistance mécanique satisfaisante, cette gélatine présentant à sa surface des sites accessibles constitués par des ligands aptes à se combiner de façon réversible avec un anti-corps ou une substance biologiquement active.

De tels supports ou membranes en gélatine de

peau de porc modifiée de manière à permettre une liaison reversible immédiate avec le complexe-antigène-anticorps marqué n'ont pas été décrits dans la technique antérieure, ni leur mode de réalisation, alors qu'ils présentent des avantages considérables. En effet, outre son rôle de support, la gélatine, du fait de sa structure primaire modifiée sert de réservoir à oxygène. Ceci est particulièrement important, lorsque, comme celà est décrit ci-après dans le cadre de l'invention, le support en gélatine est associé à un capteur électrochimique tel qu'une électrode à $pO_2$. En effet, le support recouvre le capteur et l'on peut opérer dans des conditions indépendantes du milieu extérieur, puisque l'on mesure une consommation d'oxygène à l'intérieur de la membrane, en présence, par exemple, du glucose et de glucose oxydase utilisée comme marqueur enzymatique.

Le ligand pourra comporter, par exemple, au moins un groupe thiol apte à former une liaison disulfide soit avec un groupe thiol d'une immunoglobuline préalablement réduite, soit avec un groupe thiol d'une substance couplée à une immunoglobuline, par exemple la ribonucléase réduite.

Dans ce cas, le "décrochage" des complexes anticorps-antigènes liés au support s'effectuera par exemple à l'aide d'une solution de mercapto-éthanol ou de dithiotréitol, ou de tout autre composé apte à rompre la liaison disulfure sans affecter l'état du support modifié.

Le ligand du support protéique conforme à l'invention pourra également être constitué par une enzyme, par exemple l'aminophényl thio β galactoside (ATPG), constituant l'inhibiteur spécifique d'une seconde enzyme couplée à un anticorps, par exemple la β -galactosidase.

La séparation du support et des complexes anticorps-antigènes fixés sur celui-ci s'effectuera dans ce cas en utilisant un agent de désorption de l'enzyme couplée à l'anticorps, par exemple de l'urée, du dithiotréitol ou de la guanidine dans le cas de β -galactosidase.

Pour utilisation des supports modifiés conformes à l'invention en vue du dosage d'antigènes ou similaires, deux techniques d'un type connu en soi pourront être utilisées, à savoir la méthode dite "sandwich" et la méthode dite "par compétition".

Dans la méthode dite "sandwich", très utilisée en radio-immunologie, les anticorps spécifiques de l'antigène à doser étant immobilisés sur le support, celui-ci est ensuite immergé dans la solution d'antigènes à doser, puis, après lavage, il est mis en contact avec les anticorps correspondants "marqués" de façon appropriée, par exemple par de la glucose oxydase. L'activité glucose-oxydasique résultante de la membrane sera ensuite évaluée à l'aide d'une électrode de CLARK ou autre,équipée d'une membrane de ce support. La concentration antigénétique du milieu sera proportionnelle à l'activité glucose mesurée. Cette méthode de dosage est simple et ne nécessite aucun antigène purifié, ce qui est très avantageux,compte tenu des difficultés d'obtention et de stabilisation d'un antigène à l'état pur et des risques de contamination en cours de manipulation.

En variante, il est possible d'utiliser la méthode dite "par compétition", ainsi appelée parce que l'antigène à doser est mis en compétition avec l'antigène purifié marqué par une enzyme pour saturer les anticorps préalablement fixés sur le support thiolé. Comme dans la méthode dite sandwich, on équipera donc d'une membrane du support selon l'invention une électrode de CLARK ou autre type de capteur électrochimique pour mesurer la réaction catalysée par l'enzyme de l'antigène marqué, par exemple pour mesurer l'oxygène produit dans le cas de la ribonucléase.

Ce procédé est bien connu dans la technique, en particulier pour le dosage d'haptènes. L'antigénéticité de cet haptène est obtenue par couplage de la molécule avec une protéine inerte, éventuellement en passant par un composé intermédiaire.

L'invention a donc également pour objet une utilisation des supports modifiés définis ci-dessus pour le dosage d'antigènes ou similaires, caractérisée en ce que l'on immobilise sur une membrane dudit support des anticorps spécifiques de l'antigène à mesurer, en ce que l'on équipe de cette membrane un capteur électrochimique, en ce que l'on immerge ladite membrane dans une solution de l'antigène à doser, puis dans une solution d'anticorps spécifiques de cet antigène marqués par une enzyme et en ce que l'on mesure avec ladite électrode la réaction catalysée par ladite enzyme.

En variante, l'invention a également pour objet une utilisation des supports modifiés définis ci-dessus pour le dosage d'antigènes ou similaires, caractérisée en ce que l'on immobilise sur une membrane dudit support des anticorps spécifiques de l'antigène à mesurer, en ce que l'on équipe de cette membrane un capteur électrochimique, par exemple une électrode de CLARK, en ce que l'on immerge ladite membrane dans une solution de l'antigène à doser et d'un antigène marqué par une enzyme et en ce que l'on mesure avec ladite électrode la réaction catalysée par ladite enzyme.

On notera qu'à l'issue des opérations de dosage, dans les deux variantes, les couples antigène-anticorps doivent être décrochés du support protéique par un agent désorbant qui n'affecte pas ce support, puisque celui-ci peut être utilisé dans plusieurs cycles successifs. Par contre, l'agent désorbant pourra sans inconvénient dénaturer les enzymes servant de marqueurs, puisque ces enzymes ne seront pas réutilisées.

Pour les deux types de support modifié mentionnés ci-dessus, les essais effectués par les Demanderesses prouvent que la régénération de la membrane utilisée sur l'électrode de CLARK ou autre à l'aide d'un agent désorbant n'affecte pas de façon sensible son activité au cours d'essais successifs, ce qui présente l'avantage considérable de permettre d'utiliser un même capteur (électrode + membrane) pour le dosage d'antigènes, d'anticorps, d'haptènes, de virus etc... dans des milieux différents et d'automatiser entièrement les processus de mesure.

Deux formes de mise en oeuvre de l'invention vont être décrites ci-après en détail, en référence à la figure unique annexée, qui représente un capteur électro-enzymatique utilisable dans les procédés de

dosage conformes à l'invention.

On décrira d'abord une première forme de mise en oeuvre de l'invention, dans laquelle le support protéique comprend des groupes thiols aptos à former des liaisons disulfure avec un anticorps (immunoglobuline).

Ce support est préparé de la façon suivante :

On fait gonfler 5 g de gélatine de peau de porc dans 100 ml de tampon phosphate pH 6,8,0,1 M,pendant 1 heure. La gélatine utilisée, commercialisée par Rousselot , présente une force en gelée de 240 blooms, ce qui correspond au nombre de grammes à appliquer sur un plongeur de 1/2 seconde d'angle de diamètre pour qu'il s'enfonce de 4 mm dans une gelée à une concentration de 6,67 %, après une maturation de 16 à 18 h à 10°C ± 0,1°C (selon A ROUSSELOT, Inf. Tech., 1973, p.8).

Cette gelée est utilisée à une concentration de 5 % en poids, ce qui permet d'obtenir une bonne viscosité lors de l'hydrolyse.

On place la solution obtenue dans un bain-marie à 50°C, jusqu'à l'obtention d'une bonne solubilisation. Afin de réaliser une membrane présentant une bonne stabilité mécanique, l'on étale alors rapidement 1 ml de la solution obtenue sur un film de polypropylène de 35 $cm^2$ de surface, préalablement traité avec une solution de lauryl sulfate à 0,5 % dans du tampon phosphate pH 6,8,0,01 M, afin d'augmenter l'adhérence du film de gélatine.

On effectue ensuite une séchage à l'air ambiant jusqu'à l'obtention d'un film sec.

On procède alors à une activation (dite "tannage") de la membrane de gélatine à l'aide d'un dialdéhyde, le glutaraldéhyde.

Dans ce but, on trempe la membrane séchée dans une solution à 1 % en poids de glutaraldéhyde dans du tampon phosphate pH 5,2,0,01 M, pendant 5 minutes. On élimine ensuite l'excès de réactif par plusieurs lavages successifs à l'eau distillée. Il a été montré, en effet, que le glutaraldéhyde réagit sous forme d'un polymère insaturé, le polyglutaraldéhyde, pour donner des liaisons amines stabilisées par conjugaison (ce qui peut expliquer l'apparition d'une coloration jaune de la membrane après tannage) entre deux résidus amines de Lysine. La distribution des groupes aldéhydiques tout au long de la molécule de polyglutaraldéhyde, alliée à la relative flexibilité de cette molécule, augmente la probabilité de rencontre et donc de réaction entre la protéine à coupler et le support amine (la gélatine en moyenne contient 35 résidus amines (Lysine) sur 1000 résidus). La taille de ce polyglutaraldéhyde est fonction du pH du milieu, de la concentration en glutaraldéhyde.

On obtient donc, après cette opération de tannage au glutaraldéhyde, une membrane protéique présentant des groupes - 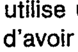 et - COOH.

Afin de thioler la membrane, on la plonge alors dans une solution 0,2 M de cystéine dans l'eau distillée. Comme agents de thiolation, on pourrait également utiliser la N acétyl homocystéine thiolactone, l'anhydride acétyl mercapto-succinique, le mercaptobutyrimidate ou le SPDP (N succinimydyl) 3-(2-pyridyl dithio)-propionate. La cystéine, de formule

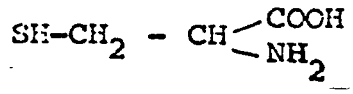

constitue cependant un agent de thiolation préféré.

On obtient ainsi une membrane thiolée , présentant des groupes -COOH et des groupes

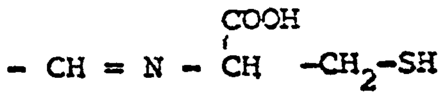

aptes à former des liaisons disulfures.

Ainsi qu'il a été indiqué ci-dessus, il est ainsi possible de fixer de façon réversible sur les groupes thiols des anticorps prélablement traités de manière à présenter eux-mêmes des groupes -SH. Il pourra s'agir d'anticorps couplés avec une enzyme présentant de tels groupes, par exemple des immunoglobulines couplées à la ribonucléase, ou encore d'anticorps ayant subi une réduction.

Pour réaliser de tels conjugés réduits, on traitera par exemple des immunoglobulines de lapin par le procédé développé par KATO, Eur. J. Biochem., 62, 285, 1976. Les groupes thiols obtenus servent à l'établissement d'une liaison disulfide avec le support . On utilisera avantageusement des tampons contenant 1 mM d'E D T A ( acide éthylènediaminetétraacétique), en vue d'augmenter la stabilité des thiols ainsi obtenus.

Pour l'application du support thiolé ainsi réalisé au dosage d'antigènes, on équipe la tête d'une électrode de CLARK à $pO_2$, du type représenté sur la figure annexée, avec une membrane réalisée à partir d'un tel support. Cette électrode comprend une micro-cathode inattaquable en platine, 1,polarisée négativement par rapport à une électrode de référence du type Ag/Ag Cl. L'électrode 1 est logée dans un corps 2 et est isolée du milieu à traiter par une membrane hydrophobe 3, par exemple en propylène, sélective aux gaz. Cette membrane 3 est appliquée contre la tête de la cathode et a un coefficient très faible de perméabilité vis-à-vis de l'oxygène. La différence de potentiel entre les électrodes est de 630 m V, ce qui correspond au courant de réduction de l'oxygène à la cathode. Ce courant est proportionnel à la pression partielle en oxygène. Les mesures sont effectuées à 25°C et le zéro de l'analyseur de $pO_2$ est réglé en utilisant une solution de dithionite.

Pour réaliser une mesure, on découpe la membrane thiolée en disques de 1 $cm^2$ de surface. Ces disques sont plongés dans une solution d'IgG (immunoglobulines) fraîchement réduits pendant une heure (on utilise un temps très long au début pour être sûr d'avoir une formation maximale de liaisons disulfures, mais, en principe, cette liaison doit s'établir rapidement).

Ces disques sont ensuitemis en contact avec 50 µl de conjugés de glucose oxydase, de faible dilution (1/10) pour avoir une réponse maximale, ceci pendant 2 h, puis on effectue un lavage soigneux.

Un tel disque 4 est ensuite placé sur le corps d'électrode 2, en contact avec la face externe de la

membrane hydrophobe 3 et l'on ferme l'assemblage de façon étanche avec un joint 5. On remplit d'électrolyte jusqu'au tiers de sa hauteur le corps d'électrode fermé par la membrane, puis on y introduit l'électrode, et l'on équipe de façon étanche son extrémité avec une cellule de mesure 6, comprenant une entrée 7 et une sortie 8 permettant la circulation à l'aide d'une pompe des différents liquides à travers la cellule.

On fait d'abord passer un tampon phosphate (pH 6.8: 0,01 M; débit 1 ml/min). On attend que le signal se stabilise, puis on fait passer le substrat (glucose à 50 g/1 dans du tampon phosphate 6,8 0,01 M). Le pH du tampon utilisé correspond à la meilleure activité de la glucose oxydase.

Quand la phase stationnaire de la cinétique enzymatique est atteinte, on fait à nouveau passer le tampon et une nouvelle mesure peut avoir lieu.

Le décrochage de la membrane du complexe Ag/Ac réduit se fera soit par une solution de mercapto éthanol, soit de dithiotréitol. Le temps de clivage et de rupture de la liaison disulfure dépendra des concentrations de ces agents.

On va décrire maintenant une seconde forme de mise en oeuvre de l'invention dans laquelle le ligand du support protéique est constitué par une enzyme, l'aminophényl thio β galactoside (en abrégé, ATPG), constituant l'inhibiteur spécifique d'une seconde enzyme, la β-galactosidase, couplée à un anticorps.

Comme dans la forme de réalisation de l'invention décrite précédemment, on utilise comme support protéique de la gélatine de peau de porc qui présente un double avantage :

(1) elle possède des fonctions susceptibles d'être utilisées dans la fixation de 1'A.T.P.G..

La gélatine est un dérivé de l'hydrolyse du collagène possédant pour 1000 résidus, 35 résidus amines et 120 résidus carboxyliques dont 35 % sont amidifiés.

L'hydrolyse basique du collagène donne naissance à de la gélatine ne conservant qu'une faible proportion de fonctions amides (pHisoélectrique acide). L'hydrolyse acide du collagène, elle, donne naissance à de la gélatine dont le pHi est proche du pHi du collagène, puisqu'une grande partie des fonctions amides est conservée.

C'est cette dernière hydrolyse que l'on utilisera ci-après. En effet, lors de la réticulation de la gélatine par le glutaraldéhyde reliant deux fonctions amines et que l'on étudiera plus loin, il est nécessaire d'avoir le maximum de fonctions amines, afin que la réticulation soit efficace.

(2) La gélatine est capable de stocker jusqu'à 25 fois plus d'oxygène que l'eau. Elle joue ainsi le rôle de réserve d'oxygène pour la réaction. Ceci permet de se libérer des contraintes de mesure liées aux variations de la concentration en $O_2$ du milieu réactionnel.

Pour préparer le support protéique, on fait gonfler 5 % de gélatine de peau de porc de dureté 250 Blooms dans de l'eau distillée pendant 1 h, à température ambiante.

Après mise à l'étuve à 40° C, jusqu'à solubilisation complète de la gélatine (1 h), on étale 1 ml de gélatine sur 35 $cm^2$ d'un film de polypropylène préalablement traité par une solution de lauryl sulfate à 0,5 % (agent tensio actif permettant une bonne adhérence de la gélatine). L'épaisseur de la membrane a été choisie de façon à obtenir une bonne réponse de l'électrode qu'elle équipera, tout en n'altérant pas les propriétés mécaniques de la membrane.

Celle-ci est alors séchée pendant 1 h à température ambiante, puis pendant 2 h sous air froid, jusqu'à obtention d'un film sec.

On procède ensuite à l'activation de la membrane de gélatine en procédant à un "tannage" par le glutaraldéhyde. Dans ce but, une solution de glutaraldéhyde à 0,5 % dans du tampon phosphate 0,01 M, pH 5,2,est étalée pendant 5 minutes sur la membrane sèche. Celle-ci est ensuite lavée abondamment par de l'eau distillée,afin d'éliminer l'excès de glutaraldéhyde n'ayant pas réagi.

Le glutaraldéhyde agit à la fois en établissant des liaisons covalentes entre les fonctions amines de la gélatine, assurant ainsi une bonne tenue mécanique de la membrane et en créant des fonctions aldéhydiques qui pourront être utilisées pour fixer l'ATPG.

On traite ensuite la membrane avec une solution de 0,1 M de glycylglycine dans 0,1 M d'acide acétique, pendant une heure, à température ambiante. On lave avec un tampon phosphate 0,01 M, pH 5,7.

On procède alors à la fixation de l'ATPG en traitant la membrane avec un mélange d'ATPG 7m M et de 1 éthyl 3(3 diméthylaminopropyl) carbodiimide 140 m M dans du tampon phosphate 0,01 M, pH 5,7, pendant 12 h à 4° C. On lave avec le même tampon phosphate.

On sature les fonctions carboxyliques activées restantes de la membrane avec de la glycine 2 M dans du tampon phosphate 0,01 M, pH 5,7, pendant 2 h, à température ambiante, puis on réduit les fonctions aldéhydiques restant sur la membrane par un borohydrure de sodium 0,M dans du tampon borate 0,1 M, pH9.

Les membranes obtenues sont conservées dans de l'eau distillée avec un agent anti-bactérien tel que de l'azoture du sodium, Na $N_3$, à 0,02 %.

L'ATPG fixé sur le support constituera l'inhibiteur spécifique d'une seconde enzyme, la β galactosidase, couplée à un anticorps qui pourra ainsi se fixer sur la membrane et avec lequel on fera réagir par compétition l'antigène à doser et un antigène marqué par la glucose oxydase.

Pour immobiliser le β galactosidase sur le support, 70 µl de β-galactosidase (Aspergillus oryzae, Sigma, 3 mg/ml et 5 u/mg) dans du tampon phosphate 0,1 M,pH 6,8,sont mis à incuber pendant 1 h sur 1 $cm^2$ de chaque type de membrane. On lave ensuite la membrane au même tampon.

On constate que la quantité de β-galactosidase immobilisée sur le support est proportionnelle à la quantité d'ATPG fixée.

Les conjugués glucose oxydase utilisés seront préparés de la manière suivante :

Pour activer les fonctions amines de la glucose oxydase par le glutaraldéhyde, 10 mg de glucose oxydase (Aspergillus niger, Sigma, 16 u/mg) sont incubés dans 1 ml de tampon phosphate 0,1 M pH

6,8 contenant 1.25 % de glutaraldéhyde, à température ambiante, pendant 18h.

On élimine l'excès de glutaraldéhyde par filtration sur gel.

La réaction de couplage est réalisée par mise en contact de la glucose oxydase activée avec 0,5 ml de NaCl 0,15 M contenant 2 mg d'anti Ac de lapin (Miles), pendant 24 h à 4°C. Les fonctions aldéhydiques présentes sur la glucose oxydase réagissent avec les fonctions amines des anti Ac.

La réaction de couplage est arrêtée par ajout de 0,5 ml de lysine 0,2 M pendant 2 h à 4°C saturant les fonctions aldéhydiques restées libres sur la glucose oxydase.

Les conjugués sont purifiés par passage de l'échantillon sur une colonne de Sephadex G200 (marque déposée) équilibrée avec du tampon phosphate 0,1 M,pH 6,8.

La membrane sur laquelle est fixé l'A.T.P.G. et immobilisée la β-galactosidase sera donc utilisée comme décrit ci-dessus en combinaison avec un capteur électrochimique, pour le dosage par la méthode dite de compétition d'un antigène en présence d'un antigène marqué, le capteur électrochimique permettant de déterminer l'activité de la glucose oxydase par mesure de l'oxygène consommé.

A la fin d'une série de mesures, le décrochage de la membrane des couples anticorps-antigène s'effectuera par lavage à l'aide d'urée 8 M.

## Revendications

1.- Support protéique modifié apte à immobiliser de façon réversible une substance biologiquement active, carctérisé en ce que ledit support comprend de la gélatine de peau de porc adhérant à un film de matière plastique ou similaire, notamment un film de polypropylène, ladite gélatine présentant à sa surface des sites accesibles constitués par des ligands aptes à se combiner de façon réversible avec un anti-corps ou une substance biologiquement active.

2.- Support selon la revendication 1, caractérisé en ce que le ligand comprend au moins un groupe thiol apte à former une liaison disulfide soit avec un groupe thiol de l'anticorps à immobiliser, notamment d'une immunoglobuline préalablement réduite, soit avec un groupe thiol d'une substance couplée avec l'anticorps à immobiliser, notamment d'une immunoglobuline.

3.- Support selon la revendication 1, caractérisé en ce le ligand comprend un substrat constituant l'inhibiteur spécifique d'une seconde enzyme couplée à l'anticorps à immobiliser.

4.- Utilisation d'un support selon l'une des revendications 1 à 3 pour le dosage d'antigènes ou similaires, caractérisée en ce que l'on immobilise sur une membrane dudit support des anticorps spécifiques de l'antigène à mesurer, en ce que l'on équipe de cette membrane un capteur électrochimique tel qu'une électrode de CLARK, en ce que l'on immerge ladite membrane dans une solution de l'antigène à doser, puis dans une solution d'anticorps spécifiques de cet antigène marqués par une enzyme, et en ce que l'on mesure avec ladite électro-que la réaction catalysée par ladite enzyme.

5.- Utilisation d'un support selon l'une des revendications 1 à 3, pour le dosage d'antigènes ou similaires, caractérisée en ce que l'on immobilise sur une membrane dudit support des anticorps spécifiques de l'antigène à mesurer, en ce que l'on équipe de cette membrane un capteur électrochimique tel qu'une électrode de CLARK, en ce que l'on immerge ladite membrane dans une solution de l'antigène à doser et d'un antigène marqué par une enzyme et en ce que l'on mesure avec ladite électrode la réaction catalysée par ladite enzyme.

6.- Utilisation selon l'une des revendications 4 et 5, caractérisée en ce que, à l'issue du dosage, les couples antigène-anticorps formés sont décrochés du support protéique par un agent désorbant n'affectant pas ledit support.

7.- Utilisation selon la revendication 6, d'un support selon la revendication 3, caractérisée en ce que ledit agent désorbant comprend une solution d'un composé apte à rompre la liaison disulfure, notamment de mercapto-éthanol ou de dithiotréitol.

8.- Utilisation selon la revendication 6 d'un support selon la revendication 3, caractérisée en ce que ledit agent désorbant comprend un agent de désorption de l'enzyme couplée à l'anticorps.

9.- Utilisation selon la revendication 8, caractérisée en ce que, dans le cas où l'enzyme liée à l'anticorps est la β-galactosidase, ledit agent désorbant est choisi dans le groupe comprenant l'urée, le dithiotréitol et la guanidine.

10.- Utilisation d'un support protéique selon l'une des revendications 1 à 3, pour la purification avec un même support de substances immunologiquement actives.

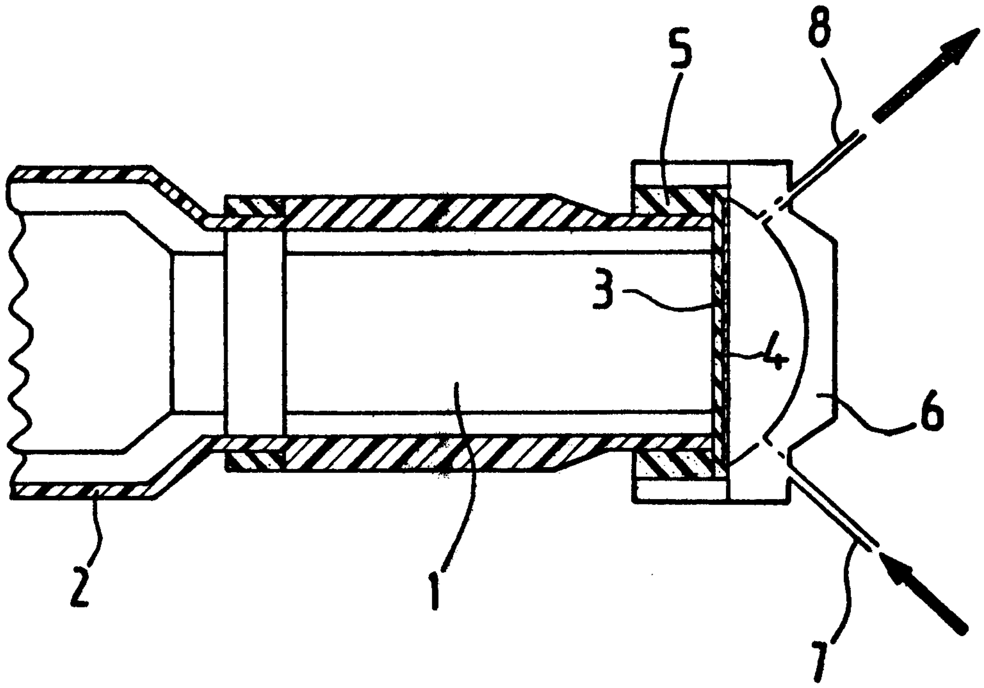
8
5
3
4
6
2
1
7

Office européen des brevets

# RAPPORT DE RECHERCHE EUROPEENNE

Numero de la demande

EP 86 40 1887

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| Y | FR-A-2 382 695 (PHARMA DIAGNOSTICS AB)<br>* Revendications 1-4; exemple 5; page 6, lignes 15,16; page 9, lignes 7-10; page 11, ligne 10 - page 12, ligne 8 * | 1,3,4 | G 01 N 33/545<br>G 01 N 33/53<br>C 12 M 1/40 |
| A | | 2 | |
| | --- | | |
| Y | FR-A-2 284 632 (UNIVERSITY OF UTAH)<br>* Revendications 1,3,13-15; page 3, lignes 10-24 * | 4 | |
| | --- | | |
| Y | US-A-4 210 418 (J.L. BROWN et al.)<br>* Colonne 3, ligne 26; colonne 3, lignes 38-47 * | 1,3 | DOMAINES TECHNIQUES RECHERCHES (Int Cl 4) |
| | --- | | G 01 N<br>C 12 M |
| Y | FR-A-2 382 696 (PHARMA DIAGNOSTICS AB)<br>* Revendications 1,2,4,5; page 3, lignes 33-35; page 3, ligne 36 - page 4, ligne 1; page 5, lignes 31-36; page 6, lignes 23,24; page 6, ligne 39 - page 7, ligne 21; page 7, ligne 39 - page 8, ligne 6; page 9, lignes 17-28; page 12, ligne 39; page 16, lignes 2-8 * | 1 | |
| | --- -/- | | |

Le present rapport de recherche a été etabli pour toutes les revendications

| Lieu de la recherche | Date d'achèvement de la recherche | Examinateur |
|---|---|---|
| LA HAYE | 31-10-1986 | COUCKE A.O.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82

Office européen des brevets

# RAPPORT DE RECHERCHE EUROPEENNE

Numero de la demande

EP 86 40 1887



## DOCUMENTS CONSIDERES COMME PERTINENTS

| Catégorie | Citation du document avec indication, en cas de besoin, des parties pertinentes | Revendication concernee | CLASSEMENT DE LA DEMANDE (Int Cl 4) |
|---|---|---|---|
| A | CHEMICAL ABSTRACTS, vol. 93, no. 25, 22 décembre 1980, page 400, résumé no. 234279v, Columbus, Ohio, US; N. YAMAMOTO et al.: "Nature of chemically modified electrodes for detection of biological substances", & NIPPON KAGAKU KAISHI 1960, (10), 1562-7 * Résumé * | 1,3,4 | |
| A | CHEMICAL ABSTRACTS, vol. 84, no. 1, 2 février 1976, page 178, résumé no. 27461x, Columbus, Ohio, US; J. CARLSSON et al.: "Reversible, covalent immobilization of enzymes by thiol-disulfide interchange", & EUR. J. BIOCHEM. 1975, 59(2), 567-72 | | |

DOMAINES TECHNIQUES RECHERCHES (Int Cl 4)

Le présent rapport de recherche a été établi pour toutes les revendications

| Lieu de la recherche | Date d'achèvement de la recherche | Examinateur |
|---|---|---|
| LA HAYE | 31-10-1986 | COUCKE A.O.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant